(19) Europäisches Patentamt
European Patent Office
Office européen des brevets

(11) Veröffentlichungsnummer: **0 362 508 A2**

(12) **EUROPÄISCHE PATENTANMELDUNG**

(21) Anmeldenummer: **89114083.2**

(22) Anmeldetag: **31.07.89**

(51) Int. Cl.5: **A61K 31/44 , A61K 7/06**

(30) Priorität: **09.08.88 DE 3826914**

(43) Veröffentlichungstag der Anmeldung: **11.04.90 Patentblatt 90/15**

(84) Benannte Vertragsstaaten: **AT BE CH DE ES FR GB GR IT LI LU NL SE**

(71) Anmelder: **Henkel Kommanditgesellschaft auf Aktien**
**Henkelstrasse 67**
**D-4000 Düsseldorf 13(DE)**

(72) Erfinder: **Giede, Karl**
**Schlehenweg 12**
**D-4010 Hilden(DE)**

(54) **Farbstabilisierung von antimikrobiellen Zubereitungen.**

(57) Bei der Einarbeitung von 1-Hydroxy-2-pyridonen in wäßrige antimikrobielle Zubereitungen auftretende, unerwünschte Farbeffekte lassen sich dadurch vermeiden, daß den Zubereitungen wasserlösliche, Sulfitionen freisetzende Salze zugegeben werden.

EP 0 362 508 A2

Xerox Copy Centre

## Farbstabilisierung von antimikrobiellen Zubereitungen

Gegenstand der Anmeldung sind wäßrige antimikrobielle Mittel mit einem Gehalt an 1-Hydroxy-2-pyridonen.

1-Hydroxy-2-pyridone, insbesondere solche Verbindungen, wie sie in den Deutschen Offenlegungsschriften 17 95 270 und 22 14 608 beschrieben wurden, sind als Substanzen mit antimikrobiellen Eigenschaften bekannt. Sie können daher als Wirkstoffe zur Bekämpfung sowohl von üblichen Hautpilzen als auch von pathogenen Keimen in entsprechenden Mitteln verwendet werden. Weiterhin ist aus der deutschen Offenlegungsschrift 22 34 009 bekannt, daß sich die genannten Verbindungen ebenfalls sehr gut als Wirkstoffe zur Bekämpfung von Kopfschuppen eignen. Wegen ihrer guten Löslichkeit und Einarbeitbarkeit in viele der gängigen Formulierungen eignen sich 1-Hydroxy-2-pyridone daher als zusätzlicher Antischuppenwirkstoff für eine große Zahl von Haarbehandlungsmitteln. Dies ist beispielsweise den Veröffentlichungen in Seifen, Öle, Fette, Wachse, 19, S 667-671, (1986) und J. Soc. Cosmet. Chem., 32, S. 327-338, (1981) zu entnehmen.

Für Produkte, die nicht ausschließlich zu therapeutischen Zwecken, sondern im Rahmen der üblichen Körperreinigung verwendet werden, ist es jedoch wichtig, daß diese nicht nur die erwarteten Eigenschaften besitzen, sondern auch in einer ästhetisch ansprechenden Formulierung angeboten werden. Wichtige Größen in diesem Zusammenhang sind optische Eigenschaften wie Farbe und Erscheinungsbild, wobei Parameter wie die Farbe durchaus auch modischen Einflüssen unterliegen.

Es hat sich nun gezeigt, daß bei der Einarbeitung von 1-Hydroxy-2-pyridonen in antimikrobielle Zubereitungen ein Farbeffekt auftritt, der in der Regel zu einem Produkt führt, das vom Konsumenten als farblich unästhetisch eingestuft wird. Dieser Effekt ist bei solchen Produkten besonders störend, bei denen durch Zugabe von Farbstoffen eine bestimmte Anfärbung des Produktes erzielt werden soll. So entstehen insbesondere bei Zubereitungen mit blauer Anfärbung häufig Produkte mit einer unansehnlichen, graugrünen Farbe. Eine Ursache für diese Farbreaktion konnte in einer Reaktion von 1-Hydroxy-2-pyridonen mit Verunreinigungen liegen, die eventuell über das verwendete Wasser oder andere Rohstoffe in das Produkt eingebracht wurden. Eine eindeutige, mechanistische Erklärung ist aber nicht bekannt.

Dieser unerwünschte Farbeffekt kann durch Zugabe von Thioglykolsäure weitgehend oder vollständig unterdrückt werden. Jedoch müssen wegen des unangenehmen Geruches der Thioglykolsäure dann weitere Maßnahmen getroffen werden, um dem Produkt eine für den Anwender akzeptable Geruchsnote zu verleihen.

Es wurde nun überraschenderweise gefunden, daß der besagte, unerwünschte Farbeffekt nicht auftritt, wenn die Mischung wasserlösliche, Sulfitionen freisetzende Salze enthält.

Gegenstand der Erfindung sind somit wäßrige antimikrobielle Mittel mit einem Gehalt an 1-Hydroxy-2-pyridonen, dadurch gekennzeichnet, daß die Mittel neben 1-Hydroxy-2-pyridonen wasserlösliche, Sulfitionen freisetzende Salze enthalten.

Es sind eine Reihe von 1-Hydroxy-2-pyridonen bekannt, die über antimikrobielle Eigenschaften verfügen. Insbesondere handelt es sich um Verbindungen der Formel,

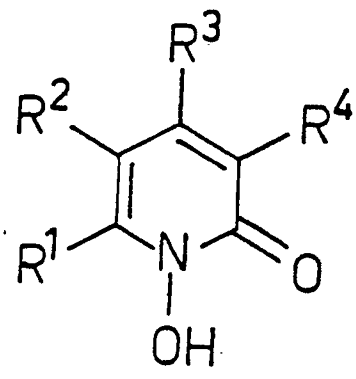

(I)

in der $R^1$ für Wasserstoff, eine Alkylgruppe mit 1-17 C-Atomen, eine Alkenylgruppe mit 2-17 C-Atomen, eine Cycloalkylgruppe mit 5-8 C-Atomen, eine Cycloalkyl-alkylgruppe, deren Alkylgruppe 1-4 C-Atome umfaßt, wobei der Cycloalkylrest durch Alkylgruppen mit 1-4 C-Atomen substituiert sein kann, eine Arylgruppe, eine Arylalkylgruppe mit 1-4 C-Atomen in der Alkylgruppe, eine Arylalkenylgruppe mit 2-4 C-Atomen in der Alkenylgruppe, eine Aryloxyalkylgruppe oder Arylmercaptoalkylgruppe mit 1-4 C-Atomen in der Alkylgruppe, eine Benzhydrylgruppe, eine Phenylsulfonylalkylgruppe mit 1-4 C-Atomen in der Alkylgruppe, eine Furylgruppe oder eine Furylalkenylgruppe mit 2-4 C-Atomen in der Alkenylgruppe steht und die vorgenannten Arylreste Phenylgruppen oder Naphthylgruppen bedeuten, die durch Alkylgruppen mit 1-4 C-Atomen, Alkoxygruppen mit 1-4 C-Atomen, Nitrogruppen, Cyanogruppen oder Halogen substituiert sein können, $R^2$ steht für Wasserstoff, eine Alkylgruppe mit 1-4 C-Atomen, eine Alkenylgruppe oder Alkinylgruppe mit 2-4 C-Atomen, eine Benzylgruppe oder Halogen, $R^3$ Wasserstoff, eine Alkylgruppe mit 1-4 Kohlenstoffatomen oder eine Phenylgruppe bedeutet und $R^4$ Wasserstoff, eine Alkylgruppe mit 1-4 C-Atomen, eine Alkenylgruppe mit 2-4 C-Atomen, eine Methoxymethylgruppe, eine Benzylgruppe oder Halogen sein kann.

Weitere geeignete Substanzen sind die Salze der genannten Verbindungen mit primären, sekundären und tertiären Aminen, Diaminen, Alkanolaminen, quartären Ammoniumhydroxiden, Guanidin und dessen Abkömmlingen. Ebenfalls geeignet sind die Salze mit anorganischen Kationen, wie die Alkalimetall-, Erdalkalimetall und Ammoniumsalze sowie die Salze mit anderen 2-, 3- und 4-wertigen Kationen.

Eine ausführliche Aufstellung dieser Verbindungen findet sich in den deutschen Offenlegungsschriften 22 34 009 und 17 95 270.

Eine als antimikrobieller Wirkstoff ganz besonders geeignete Substanz stellt das Ethanolaminsalz des 1-Hydroxy-4-methyl-6-(2,4,4-trimethylpentyl)-2(1H)-pyridons dar. Diese Verbindung ist auch unter dem Namen Piroctone Olamin bekannt und wird von der Firma Farbwerke Hoechst AG unter dem Warenzeichen OCTOPIROX(R) vertrieben.

1-Hydroxy-2-pyridone werden üblicherweise in Konzentrationen von 0,05 bis 10 Gew.-%, insbesondere von 0,05 bis 2 Gew.-%, bezogen auf das gesamte antimikrobielle Mittel, eingesetzt.

Besonders bevorzugt sind solche antimikrobiellen Mittel, die einen Gehalt von 0,1 bis 1 Gew.-% an 1-Hydroxy-2-pyridonen enthalten.

Die unerwünschten Farbeffekte bei antimikrobiellen Mittel mit 1-Hydroxy-2-pyridonen lassen sich vermeiden, indem diesen Mitteln wasserlösliche, Sulfitionen freisetzende Salze zugegeben werden. Als wasserlösliche Salze sind erfindungsgemäß solche Verbindungen zu verstehen, bei deren Zugabe zu wäßrigen antimikrobiellen Mitteln eine meßbare Erhöhung der Konzentration an Sulfitionen auftritt. Als besonders geeignet haben sich Alkalimetallsulfite, wie beispielsweise Natrium-, Kalium- und Lithiumsulfit erwiesen. Gleichfalls geeignet sind Erdalkalimetallsulfite wie Calcium- und Magnesiumsulfit. Ebenfalls erfindungsgemäß einsetzbar sind Sulfite mit anderen anorganischen Kationen, deren Verwendung in kosmetischen Mittel unbedenklich ist. Als Beispiel hierfür sei das Zinksulfit genannt. Eine weitere Gruppe erfindungsgemäß einsetzbarer Verbindungen stellen die Ammoniumsulfite dar. Unter Ammoniumsulfite werden in diesem Zusammenhang solche Sulfite verstanden, die als Kationen Ammoniumionen, Mono-, Di-, Tri- oder Tetraalkyl- oder -alkyolammoniumionen enthalten, wobei die Alkylreste 1 bis 4 C-Atome aufweisen.

Als eine ganz besonders geeignete Verbindung hat sich Natriumsulfit erwiesen.

Ebenfalls ganz besonders geeignet ist eine Mischung aus Natriumsulfit und dem Pentanatriumsalz der Aminotrimethylenphosphonsäure, das unter der Bezeichnung Turpinal(R) D 6 im Handel erhältlich ist.

Um den erfindungsgemäßen Effekt zu erhalten, werden den antimikrobiellen Mitteln die Sulfitionen freisetzenden Verbindungen in Konzentrationen von 0,01-5 Gew.-%, insbesondere von 0,01-1 Gew.-%, zugesetzt. Vor allem im Falle der Verwendung anorganischer Sulfite ist es in der Regel ausreichend, wenn diese in Konzentrationen von 0,05-1 Gew.-%, verwendet werden. Insbesondere bei der Verwendung von Natriumsulfit hat sich eine Konzentration von 0,05-0,5 Gew.-% als ausreichend erwiesen.

Ungeachtet der breiten antimikrobiellen Eigenschaften von 1-Hydroxy-2-pyridonen liegt ein Schwerpunkt ihrer Anwendung als Antischuppenwirkstoff in Haarbehandlungsmitteln. So können diese Verbindungen in einer Vielzahl von Konsumentenprodukten wie Haarshampoos, Haarwässern, Haarspülungen, Haarfestigern, Haarkuren, Haarcremes und Haarlotionen eingesetzt werden.

Diese Haarbehandlungsmittel sind üblicherweise wäßrige oder wäßrig-alkoholische Lösungen oder Dispersionen oder werden als Öl-in-Wasser- oder Wasser-in-Öl-Emulsionen formuliert. Sie enthalten dann als weitere Komponenten die in diesen Zubereitungen üblichen Verbindungen wie beispielsweise Tenside, Emulgatoren, Ölkomponenten, Fette und Wachse, Perlglanzmittel, Lösungsvermittler, Verdickungsmittel, Überfettungsmittel, Schaumstabilisatoren, biogene Wirkstoffe, Filmbildner, Duftstoffe, Farbstoffe, Konservierungsmittel und pH-Regulatoren.

Als Tenside können anionische, kationische, ampholytische, zwitterionische und nichtionogene oberflächenaktive Stoffe eingesetzt werden.

Es ist im Rahmen der erfindungsgemäßen Lehre bevorzugt, Aniontenside, gewünschtenfalls zusammen mit ampholytischen und/oder zwitterionischen Tensiden, zu verwenden. Geeignete Aniontenside sind beispielsweise Fettalkoholsulfate, Fettalkohol(polyglycol)ethersulfate, Fettalkohol(polyglycolether)-

carboxylate, α-Olefinsulfonate, Alkansulfonate, Sulfobernsteinsäuremono- und dialkylester, Acyltauride, Acylisethionate und Acylsarkoside. Besonders bevorzugt für den Einsatz in Shampoos sind aus dieser Gruppe die Fettalkoholsulfate wie beispielsweise das Triethanolammoniumlaurylsulfat, Fettalkoholethersulfate wie beispielsweise das Natriumlaurylpolyglycolethersulfat sowie Fettalkoholether(polyglycolether)carboxylate.

Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopro pylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Zwitterionische Tenside tragen im Molekül mindestens eine quartäre Ammoniumgruppe und mindestens eine-$COO^{(-)}$-oder -$SO_3^{(-)}$-Gruppe. Besonders geeignete zwitterionische Tenside sind beispielsweise die sogenannten Betaine wie N-Alkyl-N,N-dimethylammonio-glycinat, N-Acyl-aminopropyl-N,N-dimethylammonioglycinat und 2-Alkyl-3-carboxylmethyl-3-hydroxyethyl-imidazolin mit jeweils 8 bis 18 C-Atomen in der Alkyl- oder Acylgruppe.

Geeignete nichtionogene Tenside sind die Anlagerungsprodukte von 1 bis 30 Mol Ethylenoxid oder Propylenoxid an Fettalkohole, Fettsäuren, Fettamine, Alkylphenole, Fettsäuremonoethanolamine, Fettsäuremono- und -diglyceride und Fettsäuresorbitanpartialester, ferner Zucker-Fettsäureester, Alkylglucoside, Alkyloligoglucoside, Methylglucosid-Fettsäurepartialester und deren Ethylenoxidanlagerungsprodukte sowie Glycerin-oxethylat-Fettsäureester. Eine weitere wichtige Klasse von nichtionogenen Tensiden sind schließlich tertiäre Aminoxide wie Kokosamidopropyl-N,N-dimethylaminoxid und N-Kokosalkyl-N,N-di-(2-hydroxy-ethyl)-aminoxid.

Als kationische Tenside können Verbindungen wie beispielsweise Alkyltrimethylammoniumchloride, Lauryldimethylammoniumchlorid, Cetylpyridiniumchlorid und Distearyldimethylammoniumchlorid eingesetzt werden.

Bei den als Tenside eingesetzten Verbindungen mit Alkylgruppen, kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Als Emulgatoren können die in kosmetischen Zubereitungen üblichen Substanzen wie z. B. Fettsäurepartialglyceride, Fettsäure-sorbitan-partialester und deren Ethoxylate, Seifen, Fettalkoholsulfate, Polyol-Fettsäureester, Fettalkoholpolyglycolether, Lanolin, Wollfettalkohole und Alkylphosphate verwendet werden.

Übliche Ölkomponenten sind Substanzen wie Paraffinöl, Pflanzenöle, Fettsäureester, Squalan und 2-Octyldodecanol, während als Fette und Wachse beispielsweise Walrat, Bienenwachs, Montanwachs, Paraffin und Cetyl-stearylalkohol Verwendung finden.

Als Lösungsvermittler werden üblicherweise niedrige ein- oder mehrwertige Alkohole wie Ethanol, Isopropanol, Ethylenglycol, Propylenglycol, Glycerin, 1,3-Butylenglycol und Diäthylenglycol verwendet.

Gebräuchliche Filmbildner sind beispielsweise Polyvinylpyrrolidon, Vinylpyrrolidon-Vinylacetat-Copolymerisate, Polymere der Acrylsäurereihe und ähnliche Verbindungen.

Als Überfettungsmittel können Substanzen wie polyethoxylierte Lanolinderivate, Lecithinderivate und Fettsäurealkanolamide verwendet werden, wobei die letzteren gleichzeitig auch als Schaumstabilisatoren in Shampoos dienen.

Geeignete Verdickungsmittel sind beispielsweise Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon und schließlich Elektrolyte wie Kochsalz und Ammoniumchlorid, gewünschtenfalls in Kombination mit Alkylethersulfaten.

Unter biogenen Wirkstoffen sind Pflanzenextrakte, Eiweißabbauprodukte und Vitaminkomplexe zu verstehen.

Als Konservierungsmittel eignen sich die in der Anlage zur Kosmetikverordnung aufgeführten Substanzen.

Als Perlglanzmittel kommen insbesondere Glycoldistearinsäureester wie Ethylenglycoldistearat, aber auch Fettsäuremonoglycolester in Betracht.

Als Farbstoffe können die für kosmetischen Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentrationen von 0,001 bis 0,1 Gew.-%, bezogen

auf das gesamte antimikrobielle Mittel, eingesetzt.

Weitere Komponenten der erfindungsgemäßen Mitteln sind gewünschtenfalls Duftstoffe und Substanzen, die der Einstellung des pH-Wertes der Mittel dienen.

Es ist bevorzugt, die erfindungsgemäße Lehre zur Stabilisierung der Farbe bei Haarbehandlungsmitteln einzusetzen, die in Form eines Shampoos konfektioniert werden. Diese enthalten bevorzugt 0,05 bis 10 Gew.-% an 1-Hydroxy-2-pyridonen und 0,01 bis 5 Gew.-% an Sulfitionen freisetzenden Verbindungen sowie wenigstens ein anionisches Tensid in einer Menge von 4 bis 20 Gew.-% in wäßrigem Medium.

Beispiel:

Das nachfolgende Rezepturbeispiel für ein Antischuppen-Shampoo dient zur weiteren Erläuterung der Erfindung, ohne diese darauf zu beschränken.

| Komponente: | | Gew.-% |
|---|---|---|
| Texapon(R) N 25[1] | | 50,0 |
| Dehyton(R) K[2] | | 10,0 |
| Lauryloctaglycolethercarbonsäure-Na-Salz (20 % Aktivsubstanz) | | 10,0 |
| Ethylenglycoldistearat | | 2,0 |
| Glucamate(R) DOE 120[3] | | 2,0 |
| Octopirox(R)[4] | | 0,7 |
| Natriumsulfit | | 0,2 |
| Turpinal(R) D 6[5] | | 0,1 |
| Sicomet Blau S[6] | | 0,005 |
| Parfümöl | | 0,5 |
| Wasser | ad | 100 |

1 28 %ige wäßrige Lösung von Alkyl($C_{12}$-$C_{14}$)-poly(2 Ethylenoxid)glycolethersulfat-Na-Salz (Fa. Henkel)

2 Kokosacylaminopropyl-N,N-dimethylammonioglycinat (30% Aktivsubstanz) (Fa. Henkel)

3 PEG(Polyethylenglycol)-120-Methylglucose-dioleat (Fa. Amerchol)

4 Ethanolaminsalz des 1-Hydroxy-4-methyl-6-(2,4,4-trimethylpentyl)-2(1H)-pyridon (Fa. Hoechst)

5 Aminotrimethylenphosphonsäure-penta-Natrium-Salz (Fa. Henkel)

6 2-[4-Diethylamino-α-(4-diethyliminio-2,5-cyclohexadienyliden) benzyl]-4-sulfobenzolsulfonat, Natriumsalz ; Colour Index 42045 (Fa. Siegle)

Im Rahmen der Herstellung eines Shampoos der genannten Formulierung bleibt bei der Zugabe des Octopirox (R) der gewünschte blaue Farbton erhalten. Dagegen tritt bei der Zugabe des Octopirox(R) zu einer analogen Formulierung, in der die Komponenten Natriumsulfit und Turpinal(R) D 6 fehlen, eine unansehnliche Verfärbung nach graugrün auf.

## Ansprüche

1. Wäßrige antimikrobielle Mittel mit einem Gehalt an 1-Hydroxy-2-pyridonen, dadurch gekennzeichnet, daß die Mittel neben 1-Hydroxy-2-pyridonen wasserlösliche, Sulfitionen freisetzende Salze enthalten.

2. Mittel nach Anspruch 1, dadurch gekennzeichnet, daß sie das Ethanolamin-Salz des 1-Hydroxy-4-methyl-6-(2,4,4-trimethylpentyl)-2(1H)-pyridons enthalten.

3. Mittel nach einem der Ansprüche 1 oder 2, dadurch gekennzeichnet, daß sie 1-Hydroxy-2-pyridone in Konzentrationen von 0,05 bis 10 Gew.-%, insbesondere von 0,05 bis 2 Gew.-%, enthalten.

4. Mittel nach einem der Ansprüche 1 bis 3, dadurch gekennzeichnet, daß sie Farbstoffe in Konzentrationen von 0,001 bis 0,1 Gew.-% enthalten.

5. Mittel nach einem der Ansprüche 1 bis 4, dadurch gekennzeichnet, daß sie 0,01 bis 5 Gew.-%, insbesondere 0,01 bis 1 Gew.-%, an wasserlöslichen, Sulfitionen freisetzenden Salzen enthalten.

6. Mittel nach einem der Ansprüche 1 bis 5, dadurch gekennzeichnet, daß sie 0,05 bis 1 Gew.-%, insbesondere 0,05 bis 0,5 Gew.-%, an wasserlöslichen, Sulfitionen freisetzenden Salzen enthalten.

7. Mittel nach einem der Ansprüche 1 bis 6, dadurch gekennzeichnet, daß sie Alkalimetall-, Erdalkalimetall- und/oder Ammoniumsulfite enthalten.

8. Mittel nach einem der Ansprüche 1 bis 7, dadurch gekennzeichnet, daß sie Natriumsulfit enthalten.

9. Mittel nach Anspruch 8, dadurch gekennzeichnet, daß sie zusätzlich das Pentanatriumsalz der Aminotrimethylenphosphonsäure enthalten.

10. Mittel nach einem der Ansprüche 1 bis 9, dadurch gekennzeichnet, daß sie zur Haarbehandlung dienen.

11. Mittel nach Anspruch 10, dadurch gekennzeichnet, daß sie in Form eines Shampoos konfektioniert sind.